# DEMANDE DE BREVET EUROPEEN

(11) **EP 4 201 299 A1**
(43) Date de publication de la demande: **28.06.2023**
(21) Numéro de dépôt: 22215867.7
(22) Date de dépôt: 22.12.2022
(51) Int. Cl.: A61B 1/00, G06F 3/00, G06K 7/14, G06F 21/36, G16H 40/63, G09F 3/00, G16H 10/60, G16H 40/20

(54) **SYSTEME DE SECURISATION DE L'UTILISATION D'UN ENDOSCOPE MEDICAL**

(30) Priorité: 22.12.2021 FR 2114204
(71) Demandeur: Axess Vision Technology, 37300 Joue les Tours (FR)
(72) Inventeur: BODIN, Vincent, 37400 Amboise (FR); DIEUDONNE, Xavier, 37250 Montbazon (FR); HALLAUER, Emmanuel, 37190 Sache (FR)
(74) Mandataire: Cabinet Beau de Loménie

(57) **Abrégé**

L'objet de l'invention concerne un système de sécurisation comportant:
- un endoscope (2) comportant un code d'identification et pourvu d'un système de vision (6),
- un appareil électronique médical (11) configuré pour transmettre le signal d'image du code d'identification du patient et le signal d'image du code d'identification de l'utilisateur, délivrés par le système de vision (6), à une base de données (15) configurée pour stocker des codes d'enregistrement patients, utilisateurs et endoscopes, agencés pour créer une chaine d'enregistrement patient, utilisateur et endoscope,
- un système de décision (17) configuré pour autoriser l'utilisation de l'endoscope lorsque les codes d'identification du patient, de l'utilisateur et de l'endoscope coïncident avec la chaine d'enregistrement patient, utilisateur et endoscope.

## Description

### Domaine Technique

La présente invention concerne le domaine technique de la sécurisation de l'utilisation d'un endoscope médical, plus précisément à usage limité, classiquement à usage unique ou multiple d'un patient.

L'endoscope médical mis en oeuvre dans le cadre de la présente invention trouve des applications particulièrement avantageuses pour permettre d'accéder à la surface interne d'un organe creux, d'une cavité ou d'un conduit naturel ou artificiel du corps humain en vue d'effectuer diverses opérations à des fins thérapeutiques, chirurgicales ou de diagnostic, et pouvant être utilisé dans le domaine des voies urinaires, des voies gastro-intestinales, du système respiratoire, du système cardiovasculaire, de la trachée, de la cavité du sinus, du système de reproduction de la femme, de la cavité abdominale ou de tout autre partie du corps humain à explorer par une voie naturelle ou artificielle.

### Technique antérieure

Classiquement, un endoscope médical est pourvu d'un système de vision permettant d'observer ou d'inspecter une partie interne du corps humain. Cet endoscope médical est destiné à être relié à un appareil électronique médical comportant une unité de traitement des signaux d'image délivrés par le système de vision de l'endoscope. Cet appareil électronique médical qui est pourvu d'un dispositif d'affichage se présente classiquement sous la forme d'un système de visualisation ou d'une tablette de commande comportant une unité de communication configurée pour communiquer avec une base de données distante faisant partie d'un établissement hospitalier dans lequel l'endoscope médical est utilisé.

Les endoscopes médicaux à usage limité permettent de réduire les coûts d'exploitation tout en limitant les pièces d'endoscopes exposées à des tissus humains. Comme tout matériel médical, l'utilisation des endoscopes médicaux à usage limité demande à être sécurisée.

Le brevet US 10 905 309 décrit un appareil d'endoscope visant à garantir une utilisation sûre et conforme à l'acte médical à réaliser. Cet appareil comporte un moyen d'identification de l'endoscope et un moyen pour déterminer si l'endoscope est adapté à un acte médical donné en utilisant l'identification de l'endoscope. Cet appareil comporte également un moyen d'invalidation qui empêche l'utilisation de l'endoscope lorsqu'il est déterminé que l'endoscope n'est pas adapté à l'acte médical donné. Un tel appareil ne permet pas de sécuriser complètement l'acte médical à réaliser à l'aide d'un endoscope médical.

Le document JP2020 081332 concerne un système d'utilisation d'un endoscope médical comportant notamment une unité de détection de connexion détectant la connexion de l'endoscope à un dispositif processeur. En plus de détecter que l'endoscope est connecté, l'unité de détection de connexion acquiert des informations d'identification pour identifier l'endoscope connecté (numéro de modèle, etc.). Ce système comporte également une unité de notification indiquant que l'endoscope dont la connexion a été détectée par l'unité de détection de connexion est l'endoscope à utiliser. Si l'endoscope connecté est différent de celui requis, un avertissement est émis.

Le système comporte également une console recevant des informations nécessaires à l'endoscopie, telles que l'identification du médecin qui réalise l'endoscopie et l'identification du patient qui fait l'objet de l'endoscopie. Par exemple, si l'identification du médecin et l'identification du patient doivent être saisis à l'aide de codes à barres, la console comprend un lecteur de codes à barres. Si l'entrée d'identification du médecin et/ou du patient depuis la console est différente de celle du médecin et/ou du patient devant utiliser l'endoscope, un avertissement à cet effet est émis. Un tel système nécessite l'utilisation d'un lecteur pour lire les informations liées au médecin et au patient. De plus, ce système n'est pas sécurisé car un simple signal d'avertissement est émis en cas de discordance entre les données.

Le document US2014/118518 décrit un système d'imagerie comportant un instrument médical séparable dont l'utilisation est contrôlée pour éviter toute contamination des patients. L'instrument médical est pourvu d'une mémoire non volatile reliée à une unité de contrôle pour permettre le transfert de données entre la mémoire non volatile et l'unité de contrôle. Cette mémoire non volatile contient des données d'identification de l'instrument médical auquel elle est associée ainsi que des données liées à l'utilisateur et l'historique de l'utilisation de l'instrument médical.

L'unité de contrôle autorise le fonctionnement de l'appareil d'imagerie uniquement si l'utilisateur de l'endoscope s'est identifié auprès de l'unité de contrôle grâce à une interface telle un clavier, une souris ou un lecteur de cartes à puce. Un tel système nécessite l'utilisation d'une interface pour sécuriser l'utilisation de l'instrument médical.

Le document JPH10155736 décrit un appareil de traitement de signal d'un endoscope ayant une fonction de traitement d'entrée de données pour entrer et afficher des informations sur le patient. A cet effet, un code à barres est attaché au dossier d'un patient et est imagé à l'aide de la caméra de l'endoscope pour être affiché sur l'écran de visualisation des images prises par l'endoscope. Le fonctionnement de cet appareil de traitement n'est pas sécurisé.

### Exposé de l'invention

La présente invention vise à remédier aux inconvénients de l'état de la technique en proposant un système de mise en oeuvre simple, adapté pour sécuriser complètement la réalisation d'un acte médical par un endoscope médical.

Pour atteindre cet objectif, l'objet de l'invention concerne un système de sécurisation de l'utilisation d'un endoscope médical comportant :
- un endoscope médical comportant un code d'identification et pourvu d'un système de vision configuré pour délivrer un signal d'image d'un code d'identification d'un patient et un signal d'image d'un code d'identification d'un utilisateur de l'endoscope médical,
- un appareil électronique médical comportant une unité de traitement du signal d'image du code d'identification d'un patient et du signal d'image du code d'identification d'un utilisateur, délivrés par l'endoscope médical, cet appareil électronique médical comportant une unité de communication configurée pour transmettre à une base de données, au moins le code d'identification, le signal d'image du code d'identification du patient et le signal d'image du code d'identification de l'utilisateur,
- une base de données configurée pour stocker des codes d'enregistrement des endoscopes médicaux, des codes d'enregistrement patients et des codes d'enregistrement utilisateurs, chaque code d'enregistrement patient étant associé à au moins un code d'enregistrement utilisateur pour créer au moins une chaine d'enregistrement patient, utilisateur et endoscope médical,
- un système de décision configuré pour autoriser l'utilisation de l'endoscope médical lorsque le code d'identification du patient, le code d'identification de l'utilisateur et le code d'identification de l'endoscope coïncident avec la chaine d'enregistrement patient, utilisateur et endoscope médical et pour interdire l'utilisation de l'endoscope médical lorsque le code d'identification du patient et/ou le code d'identification de l'utilisateur et/ou le code d'identification de l'endoscope ne coïncident pas avec la chaine d'enregistrement patient, utilisateur et endoscope médical.

Selon un exemple de mise en oeuvre, le système de vision est configuré pour délivrer un signal d'image du code d'identification de l'endoscope médical.

Selon un autre exemple de mise en oeuvre, l'endoscope médical comporte une mémoire non volatile contenant le code d'identification de l'endoscope médical.

Selon un autre exemple de mise en oeuvre, l'endoscope médical comporte un microcontrôleur apte à générer le code d'identification de l'endoscope médical.

Selon un exemple de mise en oeuvre, le microcontrôleur est configuré pour comporter un code d'activation enregistré pour un endoscope médical et pour demander à l'appareil électronique médical, un code d'activation de l'endoscope médical, l'appareil électronique médical comportant un dispositif d'acquisition d'un code d'activation d'un endoscope médical qui est transmis au microcontrôleur pour assurer l'activation de l'endoscope médical lorsque le code d'activation reçu correspond au code d'activation enregistré de l'endoscope médical.

Selon une caractéristique de l'invention, l'appareil électronique médical comporte un contrôleur du fonctionnement de l'endoscope médical, configuré pour autoriser l'utilisation de l'endoscope médical lorsque le code d'identification du patient et le code d'identification de l'utilisateur coïncident avec la chaine d'enregistrement patient et utilisateur et pour interdire l'utilisation de l'endoscope médical lorsque le code d'identification du patient et/ou le code d'identification de l'utilisateur ne coïncident pas avec la chaine d'enregistrement patient et utilisateur.

Avantageusement, l'appareil électronique médical comporte un contrôleur du fonctionnement de l'endoscope médical, configuré pour autoriser l'utilisation de l'endoscope médical lorsque le code d'identification de l'endoscope médical coïncide avec la chaine d'enregistrement patient, utilisateur et endoscope médical et pour interdire l'utilisation de l'endoscope médical lorsque le code d'identification de l'endoscope médical ne coïncide pas avec la chaine d'enregistrement patient, utilisateur et endoscope médical.

De préférence, le système de décision comporte un dispositif de commande qui lorsque le système de décision autorise l'utilisation de l'endoscope médical, est configuré pour créer un dossier médical enregistrant les informations du code d'identification du patient, du code d'identification de l'utilisateur et du code d'identification de l'endoscope médical.

Avantageusement, le dispositif de commande est configuré pour, en relation du dossier médical créé avec les informations des codes d'identification du patient, de l'utilisateur et de l'endoscope médical, enregistrer les images acquises par le système de vision.

Typiquement, l'appareil électronique médical comporte un écran d'affichage et le système de décision est configuré pour afficher sur l'écran d'affichage, l'autorisation ou non de l'utilisation de l'endoscope médical.

### Brève description des dessins

[Fig. 1] La figure 1 est une vue schématique illustrant un système de sécurisation conforme à l'invention, de l'utilisation d'un endoscope médical.
[Fig. 2] La figure 2 est un schéma bloc fonctionnel d'un exemple de réalisation d'un système de sécurisation conforme à l'invention, de l'utilisation d'un endoscope médical.
[Fig. 3] La figure 3 est un exemple d'organigramme simplifié explicitant le principe de mise en oeuvre du système de sécurisation conforme à l'invention de l'utilisation d'un endoscope médical pourvu d'un code d'identification lisible par le système de vision de l'endoscope.
[Fig. 4] La figure 4 est un organigramme simplifié explicitant une variante de l'organigramme de la figure 3 pour laquelle le code d'identification de l'endoscope médical est dans une mémoire interne de l'endoscope.
[Fig. 5] La figure 5 est un organigramme simplifié explicitant une variante de l'organigramme de la figure 3 pour laquelle le code d'identification de l'endoscope médical est généré par un microcontrôleur interne à l'endoscope.

### Description des modes de réalisation

L'objet de l'invention concerne un système de sécurisation 1 de l'utilisation d'un endoscope médical 2 au sens général, adapté pour accéder et inspecter toute partie du corps humain à partir d'une voie d'accès naturelle ou artificielle. De manière classique, l'endoscope médical 2 permet d'inspecter les tissus en surface ou en profondeur, d'un organe creux, d'une cavité ou d'un conduit du corps humain à des fins thérapeutiques, chirurgicales ou de diagnostic. L'objet de l'invention concerne plus précisément un endoscope médical à usage limité, classiquement à usage unique ou multiple d'un patient.

D'une manière classique, l'endoscope médical 2 comporte un tube d'insertion 3 présentant une extrémité libre formant une partie ou tête distale 4 et reliée du côté opposé, à une poignée de commande 5. Le tube d'insertion 3 est fixé de manière temporaire ou définitive sur la poignée de commande 5. Ce tube d'insertion 3 qui présente une longueur et une flexibilité plus ou moins importante est destiné à être introduit dans une voie d'accès naturelle ou artificielle en vue d'effectuer diverses opérations ou fonctions à des fins thérapeutiques, chirurgicales ou de diagnostic. Le tube d'insertion 3 est en contact avec les tissus, les organes humains ou des appareillages médicaux (trocarts ou sondes), relève essentiellement d'un usage unique ou multiple d'un patient voire d'un usage réutilisable après décontamination, désinfection ou stérilisation.

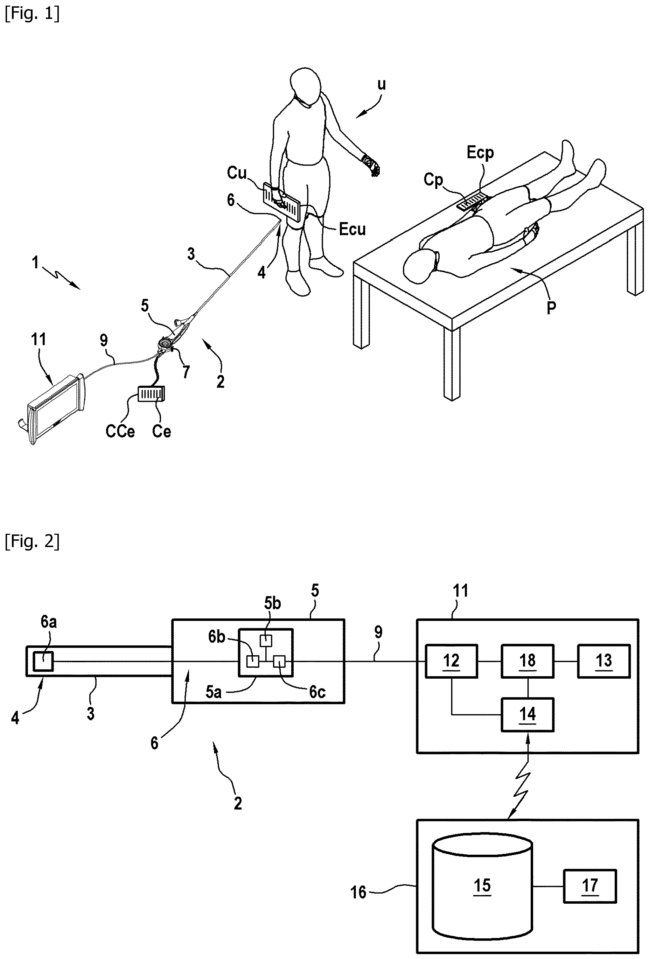

De manière classique, l'endoscope médical 2 comporte un système de vision 6 de tous types connus en soi, apte à éclairer et à ramener une image de la partie distale 4 du tube d'insertion 3. L'endoscope médical 2 comporte ainsi un système de vision 6 monté à l'intérieur de la poignée de commande 5 et pénétrant à l'intérieur du tube d'insertion 3 jusqu'à la tête distale 4. Par exemple, le système de vision 6 comporte une caméra comportant un capteur de vision 6a monté au niveau de la tête distale 4 et configuré notamment pour observer et prendre des images de la partie interne du corps humain. Le capteur de vision 6a est relié à un circuit 6b de conditionnement du signal délivré par la caméra, comme cela ressort de la figure 2. Ce circuit de conditionnement 6b est fixé sur une carte électronique 5a montée dans la poignée de commande 5. Le système de vision 6 n'est pas décrit plus précisément dans la mesure où il est bien connu de l'homme du métier.

L'endoscope médical 2 comporte également à l'intérieur du tube d'insertion 3, un canal opérateur ou de travail s'étendant de la poignée de commande 5 à la tête distale 4 pour permettre l'amenée de divers outillages et/ou de fluides et/ou l'aspiration de fluides. L'endoscope médical 1 comporte également un mécanisme de commande 7 permettant d'orienter la tête distale 4 par rapport à l'axe longitudinal du tube d'insertion 3. A cet effet, le tube d'insertion 3 comporte en amont de la tête distale 4, une structure de flexion, de pliage ou de béquillage permettant l'orientation de la tête distale 4 par rapport à l'axe longitudinal du tube d'insertion 3.

Selon une caractéristique de l'invention, l'endoscope médical 2 comporte un code d'identification Ce. Ce code d'identification Ce de l'endoscope médical contient des données d'identification de l'endoscope médical 2. Par exemple, ce code d'identification Ce de l'endoscope médical contient en tant que données, le numéro de série, les références du fabricant, la date de péremption, le type d'endoscope (cystoscope, bronchoscope, gastroscope...). Ce code d'identification Ce de l'endoscope médical peut se présenter sous différentes formes telles que par exemple, un code à une dimension ou deux dimensions, un code-barres, un QR code, un code alphanumérique ou un code numérique.

Selon une variante de réalisation, le code d'identification Ce de l'endoscope médical 2 se présente sous la forme d'un code lisible par le système de vision 6 de l'endoscope médical. Ainsi, le code d'identification Ce de l'endoscope médical 2 est imprimé sur une étiquette ou une notice CCe portée directement par l'endoscope médical (figure 1) ou par l'emballage de l'endoscope médical.

Selon autre une variante de réalisation, l'endoscope médical 2 comporte une mémoire non volatile 5b contenant le code d'identification Ce de l'endoscope médical (figure 2). Cette mémoire non volatile telle qu'une mémoire EEPROM est montée sur la carte électronique 5a logée à l'intérieur de la poignée de commande 5. Cette mémoire non volatile 5b contient notamment le code d'identification Ce de l'endoscope médical 2 auquel elle est associée.

Selon une autre variante de réalisation, l'endoscope médical 2 comporte un microcontrôleur configuré pour délivrer le code d'identification Ce de l'endoscope médical 2. Selon cette variante de réalisation, le microcontrôleur qui est pourvu d'une mémoire interne, est par exemple monté sur la carte électronique 5a qui est logée à l'intérieur de la poignée de commande 5. Ce microcontrôleur contient en particulier, le code d'identification Ce de l'endoscope médical ou un algorithme générant une clé d'accès au code d'identification Ce de l'endoscope médical comme cela sera expliqué dans la suite de la description.

L'endoscope médical 2 est relié, généralement par une liaison filaire 9, à un appareil électronique médical 11 de traitement d'informations comportant une unité de traitement 12 du signal délivré par le système de vision 6. Par exemple, cette unité de traitement 12 est reliée par la liaison filaire 9, au circuit 6b de conditionnement du signal délivré par le capteur de vision 6a, via une interface de communication 6c, comme cela ressort de la figure 2.

L'appareil électronique médical 11 de traitement d'informations comporte également un écran d'affichage 13 permettant d'afficher notamment les images acquises par le système de vision 6 et traitées par l'unité de traitement 12. L'appareil électronique médical 11 de traitement d'informations comporte classiquement, une interface homme/machine permettant à un utilisateur d'entrer des données ou de piloter cet appareil. Cette interface homme/machine peut être un clavier, une souris, un écran tactile par exemple, ou l'écran d'affichage 13. L'appareil électronique médical 11 de traitement d'informations comporte également une unité de communication 14 configurée pour communiquer avec une base de données 15, généralement distante, faisant partie d'un système informatique 16 affecté à un établissement hospitalier dans lequel l'endoscope médical 2 est utilisé. L'unité de communication 14 est apte à assurer le transfert à la base de données 15, en particulier des images acquises par le système de vision 6 et traitées par l'unité de traitement 12.

Cette base de données 15 est notamment configurée pour stocker des codes d'enregistrement patients pour des patients P devant être examinés dans l'établissement hospitalier à l'aide des endoscopes médicaux 2 et pour stocker des codes d'enregistrement utilisateurs pour des utilisateurs U des endoscopes médicaux 2. Chaque patient P possède un code d'enregistrement patient enregistré dans la base de données 15 et indiquant au moins l'identité du patient (cryptée ou non) et l'acte médical qu'il doit subir correspondant à un type d'endoscope (cystoscope, bronchoscope, gastroscope...). De même, chaque utilisateur U d'un endoscope c'est-à-dire chaque praticien ou chirurgien habilité à exercer au sein de l'établissement hospitalier, possède un code d'enregistrement utilisateur enregistré dans la base de données 15 et indiquant au moins l'identité de l'utilisateur (chiffrée ou non) et au moins un acte médical autorisé avec un type d'endoscope.

Il est à noter que la base de données 15 est configurée également pour stocker des codes d'identification des endoscopes médicaux autorisés. Chaque endoscope médical 2 possède un code endoscope enregistré indiquant le type d'endoscope autorisé à être utilisé.

Dans la base de données 15, chaque code d'enregistrement patient d'un patient P est associé à au moins un code d'enregistrement utilisateur d'un utilisateur U pour créer au moins un lien ou une chaine d'enregistrement patient et utilisateur. En d'autres termes, comme chaque code d'enregistrement patient comporte au moins l'indication de l'acte médical qu'il va subir avec un type d'endoscope médical, il est possible d'attribuer au patient, au moins un utilisateur U possédant dans son code d'enregistrement utilisateur, l'indication de l'acte médical que le patient est destiné à subir avec le type d'endoscope correspondant. Il est ainsi créé au moins une chaine d'enregistrement patient et utilisateur indiquant qu'un tel utilisateur peut intervenir sur un tel patient. Bien entendu, dans la base de données 15, il est possible de créer une chaîne entre un patient P et un seul utilisateur U ou une chaîne entre un patient et plusieurs utilisateurs.

Selon une caractéristique de l'invention, il est créé dans la base de données 15, une chaine d'enregistrement patient P, utilisateur U et endoscope médical 2. En d'autres termes, comme chaque code d'enregistrement patient comporte au moins l'indication de l'acte médical qu'il va subir avec un type d'endoscope médical, il est possible d'attribuer au patient, au moins un utilisateur U possédant dans son code d'enregistrement utilisateur, l'indication de l'acte médical que va subir le patient avec un type d'endoscope. Il est ainsi créé au moins une chaine d'enregistrement patient, utilisateur et endoscope médical indiquant qu'un tel utilisateur peut intervenir sur un tel patient avec un type déterminé d'endoscope médical. Bien entendu, dans la base de données 15, il est possible de créer une chaîne entre un patient P, un seul utilisateur U, un seul endoscope médical 2 ou une chaîne entre un patient, plusieurs utilisateurs et/ou plusieurs endoscopes médicaux.

Par ailleurs, chaque patient P comporte un code d'identification Cp contenant des données d'identification du patient. Par exemple, ce code d'identification Cp du patient contient en tant que données, l'identité du patient chiffrée ou non et au moins le type d'endoscope (cystoscope, bronchoscope, gastroscope...) devant être utilisé pour l'acte médical que le patient doit subir. Ce code d'identification Cp du patient se présente sous la forme d'un code lisible par le système de vision 6 de l'endoscope médical. Le code d'identification Cp du patient peut se présenter sous différentes formes telles que par exemple, un code à une dimension ou deux dimensions, un code-barres, un QR code ou un code alphanumérique. Typiquement, le code d'identification Cp du patient P est imprimé sur une étiquette ECp formant un bracelet.

De même, chaque utilisateur U comporte un code d'identification Cu contenant des données d'identification de l'utilisateur. Par exemple, ce code d'identification Cu de l'utilisateur U contient en tant que données, l'identité de l'utilisateur U cryptée ou non et au moins un type d'endoscope (cystoscope, bronchoscope, gastroscope...) que l'utilisateur U est habilité à utiliser pour effectuer un acte médical sur un patient. Ce code d'identification Cu de l'utilisateur U se présente sous la forme d'un code lisible par le système de vision 6 de l'endoscope médical. Le code d'identification Cu de l'utilisateur U peut se présenter sous différentes formes telles que par exemple, un code à une dimension ou deux dimensions, un code-barres, un QR code ou un code alphanumérique. Typiquement, le code d'identification Cu de l'utilisateur U est imprimé sur une carte ou une étiquette ECu par exemple portée par l'utilisateur.

Le système de sécurisation 1 conforme à l'invention comporte un endoscope médical 2 pourvu d'un système de vision 6 configuré pour délivrer un signal d'image du code d'identification Cp d'un patient P et un signal d'image du code d'identification Cu d'un utilisateur U de l'endoscope médical. En d'autres termes, le système de vision 6 de l'endoscope médical 2 est utilisé pour lire le code d'identification Cp d'un patient P et le code d'identification Cu d'un utilisateur U de l'endoscope médical. Il est à noter que dans le cas où le code d'identification Ce de l'endoscope médical 2 se présente sous la forme d'un code lisible par le système de vision 6, alors le système de vision 6 est configuré pour délivrer un signal d'image du code d'identification Ce de l'endoscope médical. Selon cette variante de réalisation, le système de vision 6 de l'endoscope médical 2 est utilisé également pour lire le code d'identification Ce de l'endoscope médical.

Le signal d'image du code d'identification Cp d'un patient, le signal d'image du code d'identification Cu d'un utilisateur, et voire le signal d'image du code d'identification Ce de l'endoscope médical, sont transmis par l'unité de communication 14, à la base de données 15.

Le système de sécurisation 1 comporte également un système de décision 17 associé à la base de données 15 et configuré pour autoriser l'utilisation de l'endoscope médical 2 lorsque le code d'identification Ce du patient et le code d'identification Cu de l'utilisateur coïncident avec la chaine d'enregistrement patient et utilisateur et pour interdire l'utilisation de l'endoscope médical lorsque le code d'identification Cp du patient et/ou le code d'identification Cu de l'utilisateur ne coïncident pas avec la chaine d'enregistrement patient et utilisateur. En d'autres termes, si l'utilisateur U est habilité à intervenir sur le patient P par ailleurs préalablement enregistré, pour accomplir l'acte médical avec un type d'endoscope, alors le système de décision 17 autorisera l'utilisation de l'endoscope médical par l'utilisateur. A l'inverse, si le code d'identification Ce du patient ne correspond pas à un patient préalablement enregistré dans la base de données et/ou si le code d'identification Cu de l'utilisateur ne correspond pas à un utilisateur autorisé pour réaliser sur le patient l'acte médical avec un type d'endoscope, alors le système de décision 17 interdit l'utilisation de l'endoscope médical 2. Le système 1 selon l'invention permet ainsi de sécuriser un acte médical avec la base de données de l'établissement hospitalier pour un patient donné en relation de l'utilisateur d'un endoscope médical, sans faire appel à un lecteur autre que l'endoscope médical.

Lorsque la chaine d'enregistrement patient, utilisateur et endoscope médical est créée, le système de décision 17 est configuré pour autoriser l'utilisation de l'endoscope médical lorsque le code d'identification de l'endoscope médical coïncide avec la chaine d'enregistrement patient, utilisateur et endoscope médical et pour interdire l'utilisation de l'endoscope médical lorsque le code d'identification de l'endoscope médical ne coïncide pas avec la chaine d'enregistrement patient, utilisateur et endoscope médical. En d'autres termes, si l'endoscope médical 2 ne correspond pas au type d'endoscope pour accomplir l'acte médical sur le patient P et/ou au type d'endoscope que peut utiliser l'utilisateur U alors le système de décision 17 interdit l'utilisation de l'endoscope médical. A l'inverse, si l'endoscope médical 2 correspond au type d'endoscope pour accomplir l'acte médical sur le patient P et au type d'endoscope que peut utiliser l'utilisateur U alors le système de décision 17 autorise l'utilisation de l'endoscope médical sur le patient par l'utilisateur. Le système 1 selon l'invention permet ainsi de sécuriser complètement un acte médical avec la base de données de l'établissement hospitalier pour un patient donné en relation d'un endoscope médical donné manipulé par un utilisateur donné.

Il est à noter que le système de décision 17 est configuré pour afficher sur l'écran d'affichage 13 l'autorisation ou non de l'utilisation de l'endoscope médical.

Selon une autre caractéristique de l'invention, l'appareil électronique médical 11 comporte un contrôleur 18 du fonctionnement de l'endoscope médical 2, piloté par le système de décision 17. Le contrôleur 18 est configuré pour autoriser l'utilisation de l'endoscope médical lorsque le code d'identification du patient et le code d'identification de l'utilisateur coïncident avec la chaine d'enregistrement patient et utilisateur et pour interdire l'utilisation de l'endoscope médical lorsque le code d'identification du patient et/ou le code d'identification de l'utilisateur ne coïncident pas avec la chaine d'enregistrement patient et utilisateur.

Selon l'invention, le contrôleur 18 est configuré pour autoriser l'utilisation de l'endoscope médical 2 lorsque le code d'identification de l'endoscope médical coïncide avec la chaine d'enregistrement patient, utilisateur et endoscope médical et pour interdire l'utilisation de l'endoscope médical 2 lorsque le code d'identification de l'endoscope médical ne coïncide pas avec la chaine d'enregistrement patient, utilisateur et endoscope médical.

Le contrôleur 18 du fonctionnement de l'endoscope médical 2 fait partie avantageusement de l'appareil électronique médical 11. Le contrôleur 18 est configuré pour autoriser ou rendre inopérant n'importe quelle(s) fonction(s) de l'endoscope médical 2. Typiquement, le contrôleur 18 est configuré pour rendre inopérant le système de vision 6. Ainsi, lorsque le système de décision 17 est configuré pour interdire l'utilisation de l'endoscope médical alors le contrôleur 18 est commandé pour par exemple, rendre inopérant le système de vision 6 de l'endoscope médical 2.

Selon une caractéristique avantageuse de mise en oeuvre, le système de décision 17 comporte un dispositif de commande qui lorsque le système de décision autorise l'utilisation de l'endoscope médical, est configuré pour créer un dossier médical enregistrant les informations du code d'identification du patient, du code d'identification de l'utilisateur et du code d'identification de l'endoscope médical. Ainsi, lorsque la chaine d'enregistrement patient et utilisateur ou la chaine d'enregistrement patient, utilisateur, endoscope médical est respectée alors les informations du code d'identification du patient, du code de l'utilisateur et voire du code de l'endoscope médical sont automatiquement enregistrées dans le dossier médical du patient dans la base de données 15. Cet enregistrement des informations évite une saisie manuelle de ces informations.

D'une manière avantageuse, le dispositif de commande est configuré pour, en relation du dossier médical créé avec les informations des codes d'identification du patient, de l'utilisateur et de l'endoscope médical, enregistrer dans la base de données 15, les images acquises par le système de vision 6 lors de la réalisation de l'acte médical par l'endoscope médical 2. Les images prises par le système de vision 6 lors de la réalisation de l'acte médical par l'endoscope médical 2 sont ainsi enregistrées dans le dossier médical associé au patient. Il apparaît une traçabilité complète tout au long de la durée de vie de l'endoscope médical 2.

La mise en oeuvre du système de sécurisation 1 conforme à l'invention découle directement de la description qui précède.

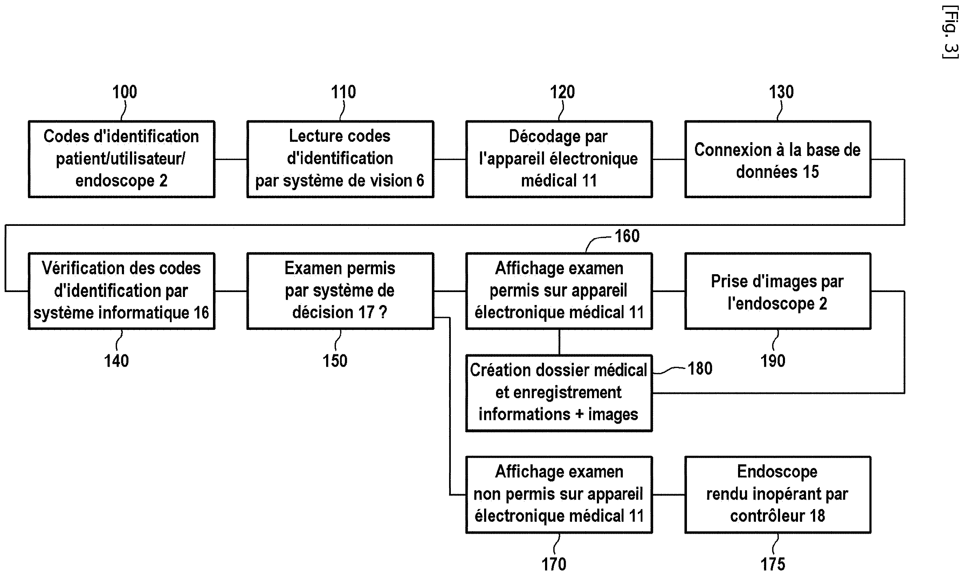

Dans l'exemple de réalisation illustré à la figure 3, il est mis à disposition un endoscope médical 2 présentant un code d'identification Ce lisible par le système de vision 6 de l'endoscope médical 2 (étape 100). Par ailleurs, le patient P est équipé d'un code d'identification Cp se présentant sous la forme d'un code lisible par le système de vision 6 de l'endoscope médical tandis que l'utilisateur U de l'endoscope médical 2 (chirurgien) est muni d'un code d'identification Cu se présentant sous la forme d'un code lisible par le système de vision 6 de l'endoscope médical 2.

Après la connexion de l'endoscope médical 2 à l'appareil électronique médical 11, et le déroulement de la phase de démarrage, l'écran d'affichage 13 de l'appareil électronique médical 11 fait apparaitre un message demandant la lecture par le système de vision 6, du code d'identification Cp du patient, du code d'identification Cu de l'utilisateur de l'endoscope médical et du code d'identification Ce de l'endoscope médical (étape 110). L'appareil électronique médical 11 assure par l'unité de traitement 12, la lecture ou le décodage des signaux d'image des codes d'identification Cp du patient, Cu de l'utilisateur et Ce de l'endoscope médical 2 (étape 120).

Après la connexion de l'appareil électronique médical 11 à la base de données 15, via l'unité de communication 14 et la transmission des codes d'identification à la base de données 15 (étape 130), le système informatique 16 vérifie les codes d'identification transmis par rapport aux codes d'enregistrement patients et utilisateurs, et la chaine d'enregistrement patient, utilisateur et endoscope médical (étape 140). A l'étape 150, le système de décision 17 effectue le test pour savoir si l'acte médical peut être réalisé sur le patient P par l'utilisateur U et avec l'endoscope médical 2, en considération des codes d'identification transmis et de la chaine d'enregistrement patient, utilisateur et endoscope médical.

Si le système de décision 17 autorise l'acte médical par l'utilisateur sur le patient et avec l'endoscope médical, alors le système de décision 17 transmet sa décision à l'appareil électronique médical 11 (étape 160) qui peut afficher sur l'écran d'affichage 13, que l'acte médical est autorisé. Si le système de décision 17 interdit l'acte médical, alors le système de décision 17 transmet sa décision à l'appareil électronique médical 11 (étape 170) qui peut afficher sur l'écran d'affichage 13, que l'acte médical n'est pas autorisé, en indiquant éventuellement les raisons de ce refus. Le contrôleur 18 rend inopérant l'endoscope médical 2 (étape 175).

Dans le cas où le système de décision 17 autorise l'acte médical, alors dans la base de données 15 est créé, un dossier médical enregistrant les informations du code d'identification du patient, du code d'identification de l'utilisateur et du code d'identification de l'endoscope médical (étape 180). Dans la mesure où l'acte médical est autorisé avec l'endoscope médical 2, l'utilisateur peut, à l'aide du système de vision 6, prendre des images (ou vidéo) de son acte médical (étape 190). Ces images prises par le système de vision peuvent être enregistrées avec le dossier médical du patient créé à l'étape 180.

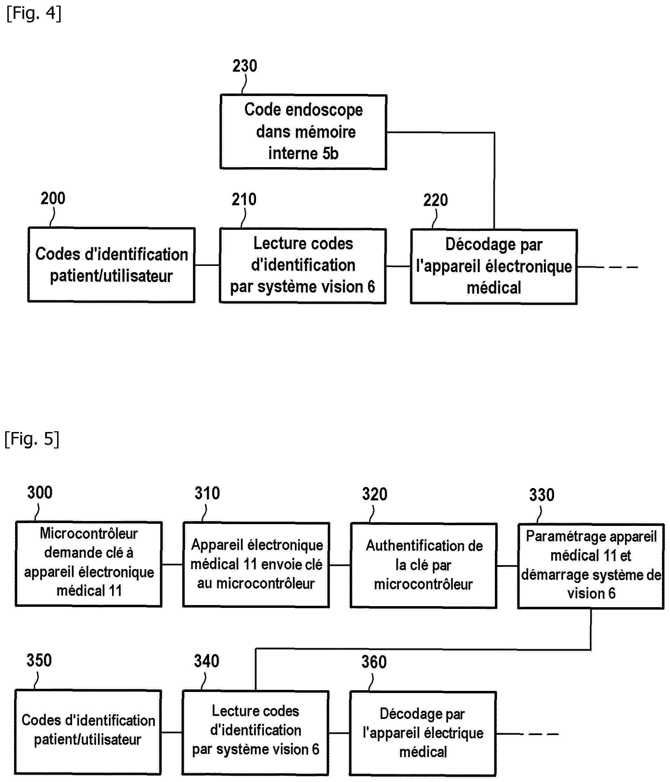

La description ci-dessus vise la variante de réalisation pour laquelle le code d'identification de l'endoscope médical est lu par le système de vision 6. La figure 4 explicite une autre variante de réalisation pour laquelle le code d'identification Ce de l'endoscope médical 2 est dans une mémoire interne 5b de l'endoscope médical (étape 230). Le patient P est équipé d'un code d'identification Cp se présentant sous la forme d'un code lisible par le système de vision 6 de l'endoscope médical tandis que l'utilisateur U de l'endoscope médical 2 (chirurgien) est muni d'un code d'identification Cu se présentant sous la forme d'un code lisible par le système de vision 6 de l'endoscope médical 2 (étape 200).

Après la connexion de l'endoscope médical 2 à l'appareil électronique médical 11, et le déroulement de la phase de démarrage, l'écran d'affichage 13 de l'appareil électronique médical 11 fait apparaitre un message demandant la lecture par le système de vision 6, du code d'identification Cp du patient et du code d'identification Cu de l'utilisateur de l'endoscope médical (étape 210). L'appareil électronique médical 11 assure par l'unité de traitement 12, la lecture ou le décodage des signaux d'image des codes d'identification Cp du patient et Cu de l'utilisateur (étape 220). De plus, l'appareil électronique médical 11 assure par l'unité de traitement 12, la prise en compte du code d'identification de l'endoscope médical 2 enregistré dans la mémoire interne 5b de l'endoscope médical (étape 230). Après cette étape de décodage, l'appareil électronique médical 11 transmet ces codes d'identification à la base de données 15 comme expliqué à la figure 3 où les étapes suivantes (à partir de l'étape 130) sont identiques aux étapes décrites en détail ci-dessus.

La figure 5 décrit une variante de réalisation pour laquelle le code d'identification de l'endoscope médical 2 est généré par un microcontrôleur monté sur la carte électronique 5a de l'endoscope médical 2. Au démarrage, le microcontrôleur demande une clé ou un code d'activation, à l'appareil électronique médical 11 (étape 300). En réponse, l'appareil électronique médical 11 envoie une clé ou un code d'activation, au microcontrôleur de l'endoscope médical (étape 310). Le microcontrôleur authentifie la clé ou le code d'activation en le comparant au code d'activation enregistré ou à la clé enregistrée dans le microcontrôleur (étape 320). Dans le cas où la clé ou le code d'activation reçu ne correspond pas au code d'activation enregistré ou à la clé enregistrée, l'endoscope médical 2 ne peut pas être utilisé.

A l'étape 330, l'appareil électronique médical 11 est paramétré et le système de vision 6 de l'endoscope médical est activé. L'écran d'affichage 13 de l'appareil électronique médical 11 fait apparaitre un message demandant la lecture par le système de vision 6, du code d'identification Cp du patient et du code d'identification Cu de l'utilisateur de l'endoscope médical (étape 340). Comme déjà expliqué, le patient P est équipé d'un code d'identification Cp se présentant sous la forme d'un code lisible par le système de vision 6 de l'endoscope médical tandis que l'utilisateur U de l'endoscope médical 2 (chirurgien) est muni d'un code d'identification Cu se présentant sous la forme d'un code lisible par le système de vision 6 de l'endoscope médical 2 (étape 350).

L'appareil électronique médical 11 assure par l'unité de traitement 12, la lecture ou le décodage des signaux d'image des codes d'identification Cp du patient et Cu de l'utilisateur (étape 360). De plus, l'appareil électronique médical 11 assure par l'unité de traitement 12, la prise en compte du code d'identification de l'endoscope médical 2 généré par le microcontrôleur de l'endoscope médical. Après cette étape de décodage, l'appareil électronique médical 11 transmet ces codes d'identification à la base de données 15 comme expliqué à la figure 3 où les étapes suivantes (à partir de l'étape 130) sont identiques aux étapes décrites en détail ci-dessus.

## Revendications

1. Système de sécurisation de l'utilisation d'un endoscope médical comportant :
- un endoscope médical (2) comporte un code d'identification (Ce) et est pourvu d'un système de vision (6) configuré pour délivrer un signal d'image d'un code d'identification (Cp) d'un patient et un signal d'image d'un code d'identification (Cu) d'un utilisateur de l'endoscope médical,
- un appareil électronique médical (11) de traitement d'informations comportant une unité de traitement (12) du signal d'image du code d'identification d'un patient et du signal d'image du code d'identification d'un utilisateur, délivrés par l'endoscope médical, cet appareil électronique médical comportant une unité de communication (14) configurée pour transmettre à une base de données (15), au moins le code d'identification (Ce), le signal d'image du code d'identification du patient et le signal d'image du code d'identification de l'utilisateur,
- une base de données (15) configurée pour stocker des codes d'enregistrement des endoscopes médicaux, des codes d'enregistrements patients et des codes d'enregistrement utilisateurs, chaque code d'enregistrement patient étant associé à au moins un code d'enregistrement utilisateur pour créer au moins une chaine d'enregistrement patient, utilisateur et endoscope médical,
- un système de décision (17) configuré pour autoriser l'utilisation de l'endoscope médical lorsque le code d'identification du patient, le code d'identification de l'utilisateur et le code d'identification de l'endoscope coïncident avec la chaine d'enregistrement patient, utilisateur et endoscope médical et pour interdire l'utilisation de l'endoscope médical lorsque le code d'identification du patient et/ou le code d'identification de l'utilisateur et/ou le code d'identification de l'endoscope ne coïncident pas avec la chaine d'enregistrement patient, utilisateur et endoscope médical.

2. Système selon la revendication 1 selon lequel le système de vision (6) est configuré pour délivrer un signal d'image du code d'identification (Ce) de l'endoscope médical.

3. Système selon la revendication 1 selon lequel l'endoscope médical (2) comporte une mémoire non volatile (5b) contenant le code d'identification (Ce) de l'endoscope médical.

4. Système selon la revendication 1 selon lequel l'endoscope médical (2) comporte un microcontrôleur apte à générer le code d'identification (Ce) de l'endoscope médical.

5. Système selon la revendication 4 selon lequel le microcontrôleur est configuré pour comporter un code d'activation enregistré pour un endoscope médical (2) et pour demander à l'appareil électronique médical (11), un code d'activation de l'endoscope médical, l'appareil électronique médical comportant un dispositif d'acquisition d'un code d'activation d'un endoscope médical qui est transmis au microcontrôleur pour assurer l'activation de l'endoscope médical lorsque le code d'activation reçu correspond au code d'activation enregistré de l'endoscope médical.

6. Système selon l'une des revendications précédentes selon lequel l'appareil électronique médical (11) comporte un contrôleur (18) du fonctionnement de l'endoscope médical, configuré pour autoriser l'utilisation de l'endoscope médical (2) lorsque le code d'identification du patient et le code d'identification de l'utilisateur coïncident avec la chaine d'enregistrement patient et utilisateur et pour interdire l'utilisation de l'endoscope médical lorsque le code d'identification du patient et/ou le code d'identification de l'utilisateur ne coïncident pas avec la chaine d'enregistrement patient et utilisateur.

7. Système selon l'une des revendications 1 à 6 selon lequel l'appareil électronique médical (11) comporte un contrôleur (18) du fonctionnement de l'endoscope médical, configuré pour autoriser l'utilisation de l'endoscope médical lorsque le code d'identification de l'endoscope médical coïncide avec la chaine d'enregistrement patient, utilisateur et endoscope médical et pour interdire l'utilisation de l'endoscope médical lorsque le code d'identification de l'endoscope médical ne coïncide pas avec la chaine d'enregistrement patient, utilisateur et endoscope médical.

8. Système selon l'une des revendications 1 à 7 selon lequel le système de décision (17) comporte un dispositif de commande qui lorsque le système de décision autorise l'utilisation de l'endoscope médical, est configuré pour créer un dossier médical enregistrant les informations du code d'identification du patient, du code d'identification de l'utilisateur et du code d'identification de l'endoscope médical.

9. Système selon la revendication précédente selon lequel le dispositif de commande est configuré pour, en relation du dossier médical créé avec les informations des codes d'identification du patient, de l'utilisateur et de l'endoscope médical, enregistrer les images acquises par le système de vision.

10. Système selon l'une des revendications précédentes selon lequel l'appareil électronique médical (11) comporte un écran d'affichage (13) et en ce que le système de décision (17) est configuré pour afficher sur l'écran d'affichage (13), l'autorisation ou non de l'utilisation de l'endoscope médical.
